# FASCICULE DE BREVET EUROPEEN

(11) **EP 1 605 997 B1**
(45) Date de publication et mention de la délivrance du brevet: **25.10.2017**
(21) Numéro de dépôt: 04742360.3
(22) Date de dépôt: 25.03.2004
(51) Int. Cl.: A61M 5/32

(54) **DISPOSITIF DE PROTECTION D AIGUILLE DESTINE A UNE SERINGUE, ET DISPOSITIF D´INJECTION COMPRENANT UNE SERINGUE ET CE DISPOSITIF DE PROTECTION.**
NADELSCHUTZVORRICHTUNG FÜR EINE SPRITZE, UND INJEKTIONSVORRICHTUNG MIT EINER SPRITZE UND DIESER NADELSCHUTZVORRICHTUNG
DEVICE FOR PROTECTION OF THE NEEDLE FOR A SYRINGE AND INJECTION DEVICE COMPRISING A SYRINGE AND SAID PROTECTION DEVICE

(30) Priorité: 25.03.2003 FR 0303657
(43) Date de publication de la demande: 21.12.2005
(73) Titulaire: Tech Group Europe Limited, Dublin 2 (IE)
(72) Inventeur: PESSIN, Olivier, F-69290 GREZIEU LA VARENNE (FR)
(74) Mandataire: Lavoix
(86) Numéro de dépôt international: PCT/FR2004/000755
(87) Numéro de publication internationale: WO 2004/087242

(56) Documents cités:
- EP-A- 0 966 983
- WO-A-01/37898
- WO-A-01/85239
- FR-A- 2 794 650
- US-A- 4 887 998
- US-A- 5 026 349
- US-A- 5 411 487

## Description

La présente invention concerne un dispositif de protection d'aiguille comportant :
- un support de protecteur délimitant un conduit de réception d'un corps de seringue ;
- un protecteur d'aiguille mobile par rapport au support de protecteur, entre une position rétractée et une position déployée ;
- un ressort de compression appliqué entre le support de protecteur et le protecteur d'aiguille ; et
- des moyens de retenue initiale du protecteur d'aiguille à l'encontre de l'action du ressort comprimé, lesquels moyens sont libérables par déplacement du protecteur d'aiguille par rapport au support de protecteur suivant une direction de libération.

Ce dispositif de protection d'aiguille comporte classiquement un support de protecteur destiné à recevoir la seringue d'injection. Le protecteur est déplaçable par rapport au support sous l'action du ressort. Des moyens de retenue initiale du protecteur par rapport au support sont prévus afin de ne permettre la libération du ressort qu'à l'issue de l'injection.

Ces moyens de retenue peuvent être libérés soit automatiquement à l'issue de l'injection, soit manuellement. Un dispositif selon le préambule de la revendication 1 est connu de WO 01/85239. Lors de leur transport, avant montage sur des seringues, les dispositifs de protection d'aiguille sont armés, c'est-à-dire que les ressorts d'actionnement des protecteurs sont comprimés. Ainsi, en cas de vibration, ou de choc sur un dispositif de protection, le ressort peut être accidentellement libéré provoquant la mise en place du protecteur d'aiguille. Le dispositif est alors inutilisable.

L'invention tel que définit dans la revendication 1 a pour but de proposer un dispositif de protection d'aiguille dont les risques de déclenchement accidentel en l'absence de seringue sont réduits.

A cet effet, l'invention a pour objet un dispositif de protection du type défini plus haut caractérisé en ce qu'il comporte des moyens mécaniques de blocage du protecteur d'aiguille par rapport au support de protecteur suivant la direction de libération, en l'absence d'un corps de seringue dans le conduit du support de protecteur, lesquels moyens mécaniques de blocage sont désactivables par engagement d'un corps de seringue dans le conduit du support de protecteur.

Suivant l'invention:
- les moyens mécaniques de blocage comportent au moins un crochet solidaire d'un premier du protecteur et du support le ou chaque crochet étant déplaçable entre une position de blocage en butée contre le second du protecteur et du support en l'absence de seringue et une position de déblocage à l'écart du second du protecteur et du support en présence d'une seringue, le ou chaque crochet présentant une face d'actionnement sous l'action du corps de seringue lors de son engagement dans le conduit du support pour le passage du crochet de sa position de blocage à sa position de déblocage.

Dans des modes de réalisation préférés :
- le ou chaque crochet présente une protubérance faisant saillie à l'intérieur du conduit du support de protecteur en l'absence du corps de seringue ;
- le ou chaque crochet est solidaire du protecteur d'aiguille ; et
- le ou chaque crochet présente une surface de came et le support de protecteur comporte au moins une rampe propre à coopérer avec la ou chaque surface de came pour assurer un effacement du ou de chaque crochet à l'extérieur du support de protecteur.

L'invention a en outre pour objet un dispositif d'injection comportant, d'une part, une seringue qui comprend une aiguille, un corps tubulaire à l'extrémité distale duquel est fixée une aiguille, et un piston d'injection monté à coulissement dans le corps, et d'autre part, un dispositif de protection d'aiguille tel que décrit ci-dessus.

L'invention sera mieux comprise à la lecture de la description qui va suivre, donnée uniquement à titre d'exemple et faite en se référant aux dessins sur lesquels :
- la figure 1 est une vue éclatée en perspective d'un dispositif d'injection selon l'invention avant assemblage ;
- la figure 2 est une vue en élévation du dispositif de protection appartenant au dispositif d'injection de la figure 1, en position rétractée ;
- la figure 3 est une vue en coupe selon le plan III-III indiqué sur la figure 2 ;
- la figure 4 est une vue de dessus selon la flèche IV indiquée sur la figure 2 ;
- la figure 5 est une vue en coupe selon le plan indiqué V-V sur la figure 4;
- la figure 6A est une vue suivant le même plan de coupe que celui de la figure 5, du dispositif d'injection selon l'invention avant utilisation ;
- la figure 6B est une vue de dessus selon la flèche VI indiquée sur la figure 6A ;
- la figure 7 est une vue analogue à celle de la figure 6A, le dispositif d'injection étant en cours d'utilisation ;
- la figure 8 est une vue suivant le même plan de coupe que celui de la figure 3, du dispositif d'injection en fin de d'utilisation ;
- la figure 9 est une vue identique à celle de la figure 8, la seringue du dispositif d'injection n'étant pas représentée ;
- la figure 10 est une vue en coupe selon le plan X-X indiquée sur la figure 8 ; et
- les figures 11 à 13 illustrent le dispositif d'injection en position déployée, la figure 11 étant une vue analogue à celle de la figure 8, la figure 12 étant une vue en coupe selon le plan XII-XII indiqué sur la figure 11 et la figure 13 est une vue identique à celle de la figure 11, la seringue n'étant pas représentée.

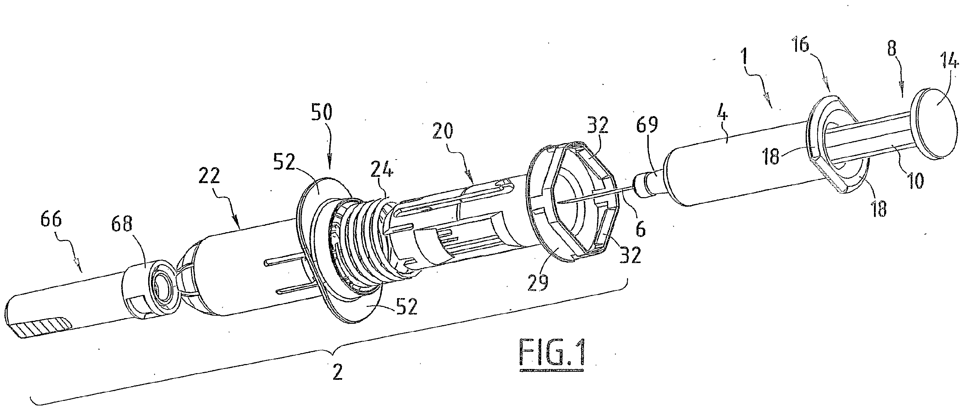

Sur la figure 1 sont représentés en perspective une seringue 1 et un ensemble de protection 2. Dans toute la suite, les termes « proximal » et « arrière » sont synonymes, tout comme les termes « distal » et « avant ».

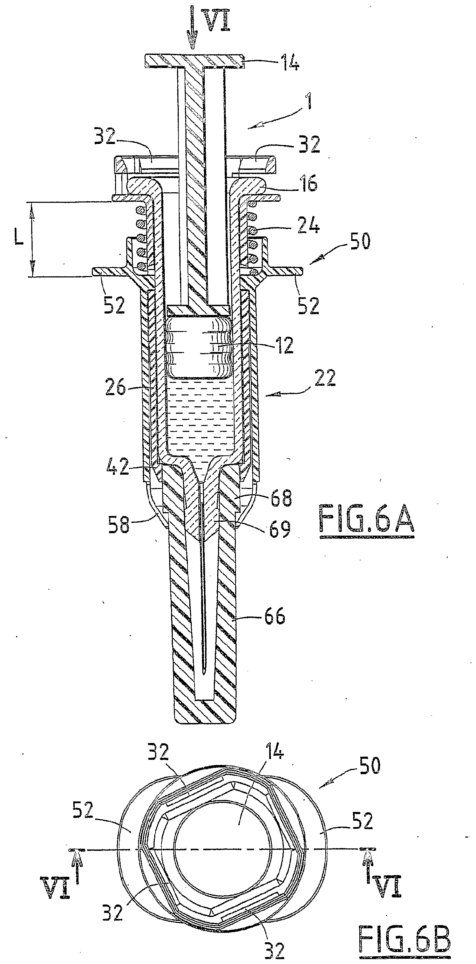

La seringue 1 est une seringue en verre de forme standard, destinée à un usage unique. Elle contient un liquide à injecter, de façon intramusculaire ou sous-cutanée, à un patient. Elle comporte à cet effet un corps 4, une aiguille 6 solidaire de l'extrémité distale du corps 4 et un piston 8. Ce piston comporte classiquement une tige 10 pourvue à son extrémité distale d'un tampon 12 visible sur la figure 6A et d'une tête d'appui 14 sur laquelle est destiné à s'appuyer le pouce de la main du praticien.

Le corps 4 de la seringue comporte dans sa partie proximale un col 16 délimitant circonférentiellement deux oreilles 18 diamétralement opposées, destinées normalement, notamment en l'absence de l'ensemble 2, à former des surfaces d'appui pour l'index et le majeur du praticien lors de la manipulation de la seringue et de l'injection du liquide s'y trouvant.

L'ensemble de protection 2, d'axe général X-X, comporte essentiellement, comme représenté sur la figure 1 :
- un support 20 de forme générale tubulaire ;
- un manchon protecteur 22 disposé coaxialement au support 20 et de diamètre supérieur à celui du support, et
- un ressort 24.

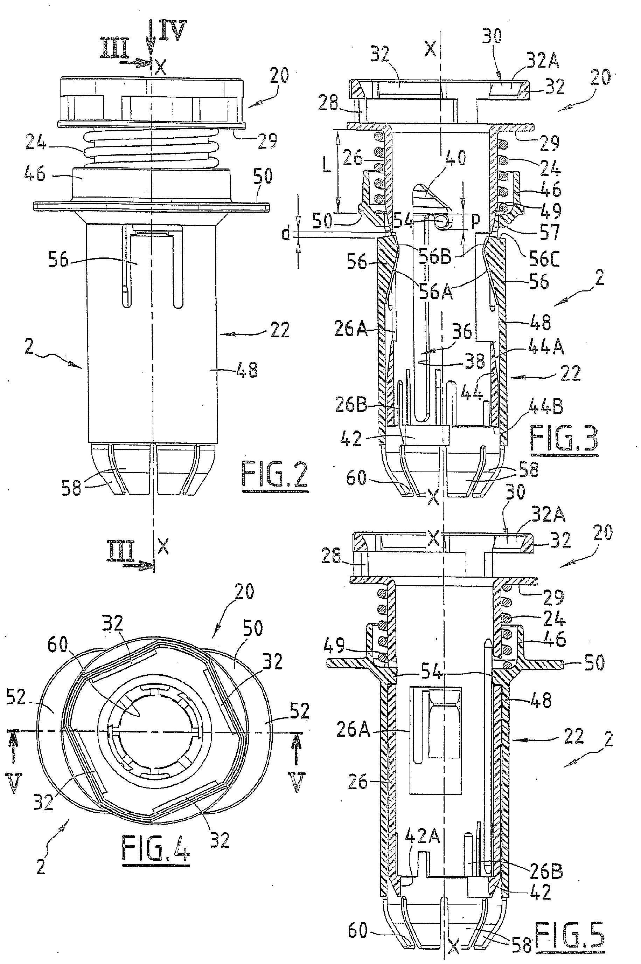

Ces trois éléments vont successivement être détaillés ci-dessous, en regard des figures 2 à 4.

Le support 20 comporte un tronçon principal 26, sensiblement cylindrique et de diamètre interne sensiblement égal au diamètre externe du corps 4 de la seringue. Ce tronçon 26 se prolonge à son extrémité proximale par un tronçon secondaire 28 de plus grands diamètres intérieur et extérieur que ceux du tronçon principal 26, en formant un épaulement radial 29.

Le tronçon proximal 28 est pourvu de moyens 30 de fixation sur le col de seringue 16. Plus précisément, ces moyens 30 comportent des crochets déformables 32 diamétralement opposés, également visibles sur la figure 1. Chacun de ces crochets ménage une surface de rampe 32A sensiblement tronconique s'évasant vers l'extrémité libre du tronçon 28, destinée à être repoussée élastiquement par les oreilles 18 du col de seringue 16 lors de la fixation du support 20 sur la seringue 1. Les crochets 32 sont distants de l'épaulement 29 d'une distance sensiblement égale à l'épaisseur des oreilles 18. Les crochets 32 forment ainsi un moyen de clipsage du col de seringue 16.

Deux rainures traversantes 36 en forme de crosse sont ménagées l'une en face de l'autre dans le tronçon principal 26. Chaque rainure est constituée d'une première partie rectiligne 38 qui s'étend sensiblement suivant l'axe X-X du support 20 sur une longueur supérieure à celle de l'aiguille 6, et d'une seconde partie rectiligne 40 qui s'étend de manière inclinée par rapport au même axe X-X. La partie inclinée 40 débouche à l'extrémité proximale de la première partie rectiligne 38, en formant un V dont la pointe est dirigée du côté proximal de l'ensemble 2.

Le tronçon principal 26 comporte à son extrémité distale une première paire de languettes élastiques 42 diamétralement opposées, situées chacune dans le prolongement des rainures 36 (figure 5). Ces languettes 42 présentent une face interne 42A de forme sensiblement cylindrique et une face externe 42B sensiblement tronconique, divergente vers l'arrière.

Le tronçon principal 26 comporte une paire de rampes externes 44 diamétralement opposées, situées entre les languettes élastiques 42 en suivant la circonférence de l'extrémité distale de ce tronçon. Elles présentent une surface externe inclinée 44A sensiblement tronconique, divergente vers l'avant, et une surface distale 44B sensiblement plane. Les surfaces externes 44A sont ainsi tournées vers les évidements 26A.

Des évidements longitudinaux 26A sont ménagés dans le tronçon principal 26 (figure 3) à l'extrémité proximale de ces rampes 44.

Par ailleurs, de part et d'autre des languettes 42, des fentes axiales 26B sont prévues à partir de l'extrémité distale du support, de sorte que, avant insertion du corps 4 de la seringue 1 à l'intérieur du support 20, ces languettes 42 sont radialement déformables, notamment vers l'intérieur.

Le manchon protecteur 22 est de longueur sensiblement égale à celle du corps 4 de la seringue 1. Il est constitué de deux tronçons cylindriques 46 et 48, le tronçon proximal 46 étant de diamètre légèrement supérieur à celui du tronçon principal 48. Ces deux tronçons se raccordent en formant un épaulement radial 49.

Le manchon 22 comporte solidairement, dans sa partie proximale, une collerette extérieure 50 sous forme de deux oreilles 52 diamétralement opposées (figure 4).

Egalement dans sa partie proximale, mais à l'intérieur du manchon protecteur 22, deux tétons 54 diamétralement opposés sont solidaires du manchon (figure 5). Ces deux tétons sont reçus et guidés dans respectivement les deux rainures 36 du support 20. Le support et le manchon sont ainsi mobiles l'un par rapport à l'autre en translation suivant leur axe commun et en rotation limitée autour du même axe lorsque les tétons se trouvent dans les parties inclinées 40. Les parties inclinées 40 forment alors des poches de rétention des tétons 54, ces poches ayant une profondeur de rétention notée p sur la Figure 3. Cette profondeur est mesurée suivant l'axe du protecteur.

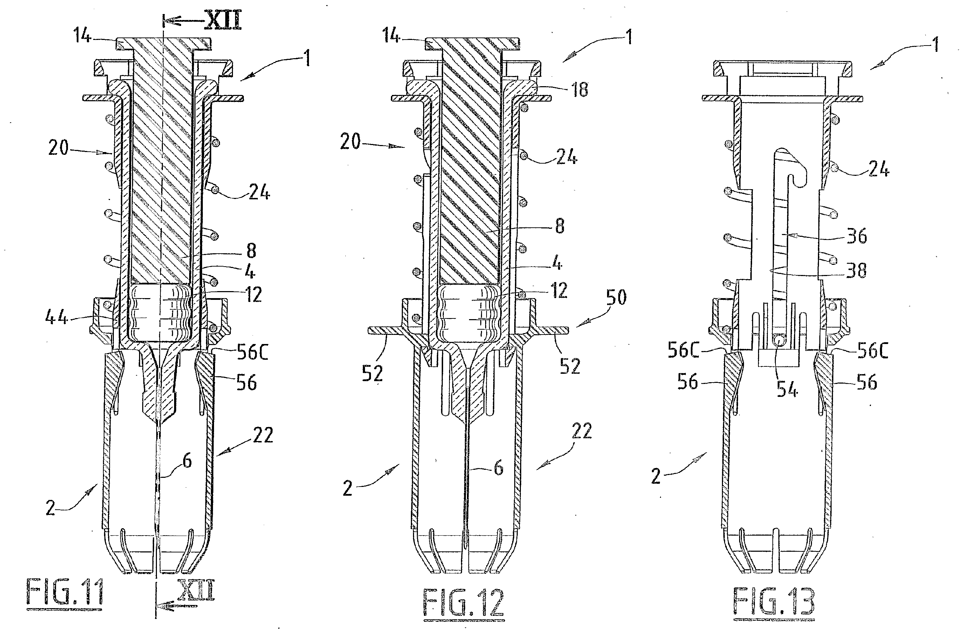

Plus précisément, le support 20 et le manchon 22 sont mobiles entre une position rétractée du manchon, dans laquelle l'essentiel du manchon recouvre l'essentiel du support et les tétons 54 sont situés à l'extrémité distale de chacune des parties de rainures inclinées 40, comme représenté sur les figures 2 à 7, et une position déployée du manchon dans laquelle celui-ci s'étend axialement en saillie du support et les tétons sont situés à l'extrémité distale de la partie de rainure rectiligne 38, comme représenté sur les figures 11 à 13.

Lorsque la seringue 1 est fixée sur l'ensemble 2, ces positions extrêmes correspondent respectivement à une configuration d'injection dans laquelle l'aiguille 6 de la seringue 1 est dégagée et destinée à être insérée dans un patient, et à une configuration de protection dans laquelle cette aiguille est entourée du manchon protecteur 22.

La partie proximale du manchon 22 comporte en outre intérieurement une paire de crochets longitudinaux déformables 56 diamétralement opposés. Ces crochets sont délimités dans le manchon 22 par des fentes latérales. Ces crochets sont liés au manchon à leur extrémité distale. Chaque crochet présente à son extrémité libre proximale une protubérance intérieure.

En l'absence de seringue, et comme illustré sur la Figure 3, les surfaces externes des crochets 56 s'étendent dans le prolongement du manchon. Au contraire, les protubérances internes de ces crochets font saillie à l'intérieur du passage cylindrique délimité par le manchon 22. Chaque protubérance présente une face avant 56A de forme sensiblement tronconique s'écartant vers l'avant de l'axe du manchon 22. Ces faces avant 56A sont ainsi tournées vers l'extrémité avant du protecteur.

Chaque face avant 56A est adaptée pour coopérer avec une surface inclinée 44A formée par les rampes 44 du support.

En outre, chaque protubérance intérieure présente une face arrière inclinée 56B tournée vers l'extrémité arrière du protecteur, et en particulier vers les deux oreilles 52. Ces surfaces 56B sont généralement tronconiques et divergent l'une de l'autre en direction de l'extrémité arrière du protecteur.

Le support 20 comporte des rampes tronconiques 57 disposées à l'arrière des évidements 26A. Ces rampes sont inclinées vers les évidements 26A et sont adaptées pour coopérer avec les faces inclinées arrières 56B.

A son extrémité libre, chaque crochet 56 présente un front transversal incliné 56C formant butée.

En l'absence de seringue, tel qu'illustré sur la Figure 3, le front 56C de chaque crochet s'étend immédiatement en regard de l'extrémité correspondante de l'évidement 26A. La distance d séparant la face d'extrémité 56C du bord de l'évidement est inférieure à la profondeur p de la poche de rétention formée de la partie de rainure inclinée 40 dans laquelle est reçu le pion 54.

Dans la position rétractée du manchon, les crochets 56 s'étendent à l'intérieur des évidements 26A ménagés dans le support 20. Dans la position déployée du manchon, comme représenté sur la figure 11, les faces d'extrémité 56C des crochets 56 sont en butée axiale contre les languettes 44, les crochets et languettes formant de la sorte un ensemble de blocage rigide en position déployée.

Le manchon 22 est par ailleurs pourvu à son extrémité distale d'une couronne de languettes déformables 58, dont les bords distaux forment une ouverture 60 sensiblement circulaire, de diamètre inférieur au diamètre intérieur du tronçon principal 26 du support 20.

Le ressort 24 est un ressort spiralé, disposé entre le manchon protecteur 22 et le support de protecteur 20. Plus précisément, le ressort est logé entre l'épaulement 29 du support 20 et l'épaulement 49 du manchon 22. Dans la position rétractée du manchon, le ressort 24 est dans un état comprimé, possédant ainsi une énergie de décompression liée à la raideur du ressort et à la différence entre la longueur du ressort à l'état au repos et sa longueur, notée L sur les figures 2 à 7, à l'état comprimé. Cela revient à dire que le ressort 24 présente un seuil de force de compression supplémentaire qui correspond à la force minimale nécessaire pour comprimer davantage le ressort à partir de son état comprimé initial des figures 2 à 7. La raideur du ressort et/ou la longueur de compression initiale L sont choisies de telle sorte que ce seuil de force soit supérieur à l'effort de poussée nécessaire pour déplacer le piston 8 de la seringue 1 sur toute sa course d'injection. Plus précisément, la force du ressort à l'état verrouillé est supérieure à la somme de l'effort d'injection, c'est-à-dire d'évacuation du liquide à l'extérieur de l'aiguille 6 de la seringue 1, et des efforts de décollement et de glissement du poussoir 12 à l'intérieur du corps 4 de la seringue.

En option, l'ensemble de protection 2 comporte en outre un capuchon 66 de forme générale tubulaire, représenté sur les figures 1 et 6A.

Le capuchon 66 est adapté pour entourer l'aiguille 6 avant utilisation de la seringue 1. Ce capuchon est fermé à une de ses extrémités et son extrémité opposée est formée par une bague annulaire 68 de diamètre extérieur adapté pour à la fois être conjugué de la surface 42A des crochets 42 du support 20 et être supérieur au diamètre de l'ouverture 60 formée par les languettes 58 du manchon protecteur 22. La face intérieure de cette bague 68 est destinée à adhérer sur la base en verre 69 du corps de seringue 4 où est fixée l'aiguille 6, notamment pour garantir une certaine étanchéité aux bactéries.

Le fonctionnement du dispositif d'injection selon l'invention est le suivant :
Le dispositif de protection 2 est assemblé dans sa configuration rétractée, c'est-à-dire celle des figures 2 à 7. Le manchon protecteur 22 est pour cela enfilé autour du support 20 à partir de l'extrémité distale du support, en disposant entre eux le ressort 24. Plus précisément, on déplace axialement vers l'arrière le manchon par rapport au support, tout en déformant radialement vers l'extérieur les crochets 56 au moyen d'un outil adapté, et ce au moins jusqu'à ce qu'ils atteignent axialement la partie avant des évidements longitudinaux 26A. Puis toujours en déplaçant le manchon vers l'arrière, les pions 54 sont appliqués contre les surfaces externes 42B des languettes 42, en déformant ces dernières vers l'intérieur, jusqu'à ce que les pions soient reçus dans les parties de rainures 38. Le déplacement du protecteur 22 vers l'arrière se poursuit ensuite jusqu'à ce que les tétons 54 soient reçus dans les parties de rainures inclinées 40, en faisant pivoter l'un par rapport à l'autre le support et le protecteur. Le protecteur se retrouve ainsi en position rétractée.

Dans cette position, et telle qu'illustré sur la Figure 3, les protubérances intérieures des crochets 56 font saillie à l'intérieur du passage de réception de la seringue de sorte que la surface transversale arrière 56C s'étend immédiatement en avant du bord correspondant de l'évidement 26A.

Le front d'extrémité forme une butée propre à coopérer avec le bord des évidements 26A pour éviter un recul du manchon protecteur 22 vers l'extrémité arrière du support 20. Les crochets 56 sont alors en position de blocage.

Comme sera expliqué ultérieurement, dans la mesure où la retenue du ressort assurant le déplacement du manchon protecteur est libérée par déplacement du protecteur vers l'arrière, tout risque de déclenchement accidentel du manchon protecteur lors du transport du dispositif de protection est évité puisque le manchon protecteur est bloqué dans sa direction de libération de manière indépendante des moyens de retenue du ressort, tant qu'aucune seringue n'est introduite dans le support de protecteur.

La seringue en verre 1 est préremplie d'un liquide à injecter dans un patient. Cette seringue est équipée du capuchon 66 qui enserre la base 67 du corps de seringue 4.

La seringue et le capuchon sont insérés à l'intérieur de l'ensemble 2 pour former le dispositif d'injection, tel que représenté sur les figures 6A et 6B. Plus précisément, le corps 4 de la seringue est déplacé sensiblement axialement à l'intérieur du support 20. Lors de l'engagement du corps 4 de la seringue dans le support 20, et comme illustré sur la Figure 6A, le corps de seringue appuie sur les faces inclinées arrières 56B, provoquant ainsi l'effacement vers l'extérieur des protubérances des crochets 56.

En particulier, les faces d'extrémité 56C des crochets et les bords correspondants des évidements 26A sont désalignés. Les crochets sont alors en position de déblocage.

Le déplacement du corps de seringue dans le protecteur est effectué jusqu'à ce que le col de seringue 16, indexé par les excroissances 34, s'enclipse à l'intérieur du tronçon proximal 28, en déformant radialement vers l'extérieur les crochets 32. Les oreilles 18 du col de seringue 16 sont alors retenues axialement par les crochets 32, tandis que les excroissances périphériques 34 retiennent le col de seringue, et donc la seringue, en rotation par rapport au support 20.

De plus, dans la mesure où le col de seringue 16 est totalement enclipsé à l'intérieur du tronçon 28, il ne peut plus remplir son rôle habituel de formation de surface d'appui pour l'index et le majeur du praticien. Cette fonction d'appui est réalisée par la collerette 50 solidaire du manchon 22. En effet, dans la mesure où la longueur du manchon protecteur 22 est sensiblement égale à celle du corps de seringue 4 et/ou où la collerette 50 est ménagée au niveau de l'extrémité proximale de ce manchon, le praticien peut alors manipuler la seringue en faisant reposer son pouce sur la tête d'appui 14 du piston 8 comme à l'accoutumée, et en faisant reposer son index et son majeur sur les faces des oreilles 52 dirigées vers l'aiguille 6.

Par ailleurs, lorsque la seringue 1 est fixée sur le support de protecteur 20 comme représenté sur les figures 6A et 6B, le capuchon 66 s'étend en saillie à l'extérieur du manchon protecteur 22.

Lorsque le praticien est prêt à réaliser l'injection du liquide contenu dans la seringue, il retire le capuchon 66 en le tirant axialement vers l'avant. La bague 68 franchit alors l'ouverture 60 en déformant les languettes 58. Une fois le capuchon 66 retiré, les languettes 58 reprennent leur position initiale. Le trou de passage 60 à l'extrémité distale du manchon, de diamètre plus petit que le diamètre extérieur du capuchon 68, empêche alors la remise en place du capuchon autour de l'aiguille. La coopération du capuchon 66 et des languettes 58 forment ainsi un moyen de contrôle de la première utilisation du dispositif d'injection.

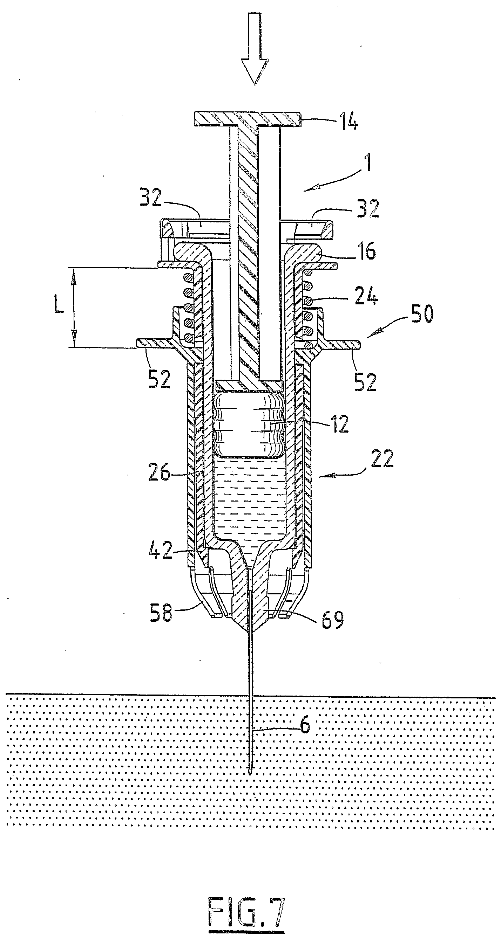

Le praticien pique ensuite l'aiguille 6 dans un patient. Il injecte le liquide contenu dans la seringue en exerçant une poussée sur la tête d'appui 14 du piston 8, son index et son majeur demeurant en contact avec les faces dirigées vers l'aiguille des oreilles 52. Durant l'injection, aucun mouvement ne se produit entre le support de protecteur 20 et le manchon protecteur 22, le ressort 24 demeurant comprimé avec une longueur L, comme représenté sur la figure 7.

L'injection se poursuit jusqu'à ce que le poussoir 12 du piston 8 parvienne en fin de course d'injection.

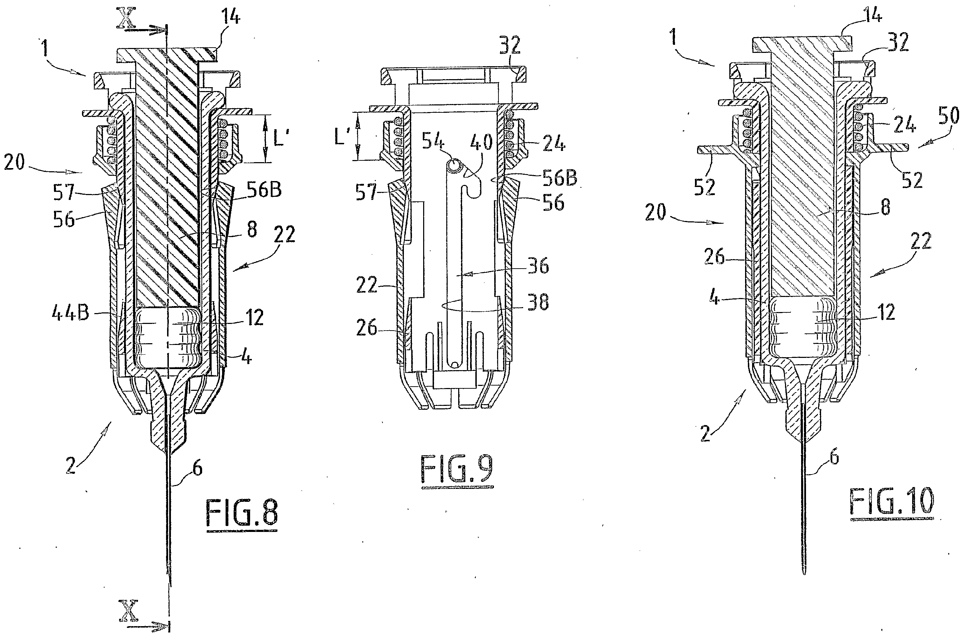

Le praticien retire alors l'aiguille du patient. Pour déclencher l'ensemble de protection 2, le praticien exerce une pression supplémentaire sur la tige de piston 8. Cette pression doit être supérieure à la force prédéterminée produite par le ressort 24 à l'état verrouillé, de sorte que ce ressort est davantage comprimé et passe de sa longueur L à une longueur L' plus petite, comme représenté sur les figures 8 à 10. Pour ce faire, en raisonnant à support de seringue immobile, le manchon protecteur 24 se déplace axialement vers l'extrémité proximale du support 20. Le praticien réalise ce mouvement en exerçant une pression correspondante au moyen de son index et de son majeur sur les oreilles 52 de la collerette 50 du manchon 22. Cette pression entraîne, de façon combinée au mouvement de translation, la rotation du manchon protecteur 22 autour du support de seringue 20, les tétons 54 étant guidés par les parties de rainure inclinées 40. Ce mouvement de rotation se poursuit jusqu'à ce que les tétons atteignent l'extrémité proximale de cette partie de rainure 40, c'est-à-dire l'extrémité proximale de la partie de rainure longitudinale 38, comme visible sur la figure 9. Le dispositif 2 est alors en position de déverrouillage du ressort 24.

Le mouvement du protecteur d'aiguille vers l'extrémité arrière du support suivant la direction de libération du ressort est rendu possible par la coopération des faces inclinées arrières 56B des protubérances des crochets et des rampes 57. Lors du mouvement du protecteur, une déformation accrue des crochets vers l'extérieur se produit comme illustré sur la Figure 8. Ainsi, les crochets s'écartent l'un de l'autre pour passer au-dessus de la surface extérieure du manchon protecteur au-delà des évidements 26A.

Le praticien relâche ensuite la pression qu'il exerçait jusqu'alors sur la collerette 50, permettant au ressort 24 de se détendre jusqu'à un état de repos. Les tétons 54 se déplacent en translation à l'intérieur de la partie de rainure longitudinale 38, jusqu'à l'extrémité distale de celle-ci comme représenté sur la figure 13. Le mouvement de translation du manchon protecteur 22 par rapport au support 20 est contrôlable par le praticien, si ce dernier relâche progressivement la retenue digitale qu'il exerce la collerette 50. Lorsque les tétons 54 sont arrivés à l'extrémité distale de la rainure 36 (figure 13), le protècteur est dans sa position déployée.

Par ailleurs, lorsque le manchon protecteur 22 est en mouvement de translation par rapport au support 20, les crochets 56 empruntent les évidements longitudinaux 26A du support, jusqu'à ce qu'ils glissent le long des rampes distales 44 du support, par coopération de leurs surfaces complémentaires 56A et 44A.

En position déployée du protecteur, les crochets 56 sont maintenus par coopération des surfaces 56C et 44B, de sorte que le manchon protecteur 22 ne peut pas être ramené dans sa position initiale. De même, le manchon 22 ne peut pas être facilement arraché du support 20 puisque les tétons 54 sont en butée contre le fond distal de la partie de rainure longitudinale 38 (figure 13), les languettes 42 formant ce fond étant radialement maintenues entre le corps de la seringue 1 et le manchon protecteur 22.

Le dispositif d'injection selon l'invention est ainsi simple d'utilisation, tout en permettant au praticien de contrôler le mouvement de recouvrement de l'aiguille par le manchon protecteur. Le nombre de pièces dont est constitué l'ensemble de protection 2 représenté est réduit à trois.

Le dispositif selon l'invention est adaptable à différents types de seringues, tant au niveau des formes qu'à celui des volumes. Ce dispositif présente donc l'avantage de ne pas remettre en cause la forme générale des seringues utilisées et par conséquent n'entraîne aucune modification des procédés industriels de remplissage de ces seringues.

Diverses variantes du dispositif selon l'invention sont envisageables :
- à la différence de l'exemple de réalisation décrit ci-dessus, les tétons 54 et/ou la collerette 50 du manchon protecteur 22 peuvent être rapportés sur le manchon 22 et non réalisés d'un seul tenant avec celui-ci ; et/ou
- les excroissances 34 des moyens 30 de fixation du support 20 sur le col de seringue 16 peuvent être supprimées et éventuellement remplacées par un ou plusieurs crochets analogues aux crochets 32 décrits ci-dessus, l'ensemble de ces crochets retenant axialement le col 16 par rapport au support 20 pour toute position angulaire relative entre la seringue 1 et le support ; dans ce cas, la seringue 1 est libre de tourner à l'intérieur du support 20, ce qui assure en contre-partie une plus grande facilité de fixation de l'ensemble de protection 2 sur la seringue (absence d'indexage).

## Revendications

1. Dispositif de protection d'aiguille comportant :
- un support de protecteur (20) délimitant un conduit de réception d'un corps de seringue (4) ;
- un protecteur d'aiguille (22) mobile par rapport au support de protecteur (20), entre une position rétractée et une position déployée ;
- un ressort de compression (24) appliqué entre le support de protecteur (20) et le protecteur d'aiguille (22) ; ledit dispositif étant **caractérisé par**:
- au moins un téton (54) du protecteur d'aiguille (22) et au moins une rainure (36) correspondante du support de protecteur (20), recevant et guidant ledit téton,
l'au moins une rainure comprenant : une première partie rectiligne (38) qui s'étend sensiblement suivant un axe du support de protecteur (20) sur une longueur supérieure à une longueur de l'aiguille à protéger ; et une seconde partie rectiligne (40) qui s'étend de manière inclinée par rapport audit axe du support de protecteur (20), les première et seconde parties (38, 40) formant un V dont une pointe est dirigée vers une extrémité proximale du dispositif,
ledit au moins un téton (54) et ladite au moins une rainure (36) étant configurés pour la retenue initiale du protecteur d'aiguille (22) à l'encontre de l'action du ressort comprimé (24) dans la position rétractée, dans laquelle le au moins un téton (54) est retenu dans la seconde partie rectiligne (40) de l'au moins une rainure (36) correspondante,
le protecteur d'aiguille (22) étant libérable par un déplacement du protecteur d'aiguille (22) par rapport au support de protecteur (20) suivant une direction de libération, conduisant au déplacement de l'au moins un téton (54) dans ladite seconde partie rectiligne (40) jusqu'à la première partie rectiligne (38) de l'au moins une rainure (36) correspondante; et
- au moins un crochet (56) solidaire d'un premier du protecteur (22) et du support (20), le ou chaque crochet étant déplaçable entre une position de blocage en butée contre le second du protecteur (22) et du support (20) en l'absence de seringue dans le support de protecteur (20) et une position de déblocage à l'écart du second du protecteur (22) et du support (20) en présence d'une seringue dans le support de protecteur (20), le ou chaque crochet (56) présentant une face (56B) d'actionnement qui coopère avec le corps de seringue (4) lors de son engagement dans le conduit du support (20) pour le passage du crochet (56) de sa position de blocage à sa position de déblocage.

2. Dispositif de protection d'aiguille suivant la revendication 1, **caractérisé en ce que** le ou chaque crochet (56) présente une protubérance faisant saillie à l'intérieur du conduit du support de protecteur (20) en l'absence du corps de seringue (4).

3. Dispositif de protection d'aiguille suivant l'une quelconque des revendications 1 et 2, **caractérisé en ce que** le ou chaque crochet (56) est solidaire du protecteur d'aiguille (22).

4. Dispositif de protection d'aiguille suivant la revendication 3, **caractérisé en ce que** le ou chaque crochet (56) présente une surface de came (56B) et le support de protecteur (20) comporte au moins une rampe (57) propre à coopérer avec la ou chaque surface de came (56B) pour assurer l'effacement du ou de chaque crochet (56) à l'extérieur du support de protecteur (20).

5. Dispositif de protection d'aiguille selon l'une quelconque des revendications 1 à 4, **caractérisé en ce que** le support de protecteur (20) comporte une surface distale (44B) propre à coopérer avec le ou chaque crochet (56) pour maintenir le protecteur (20) en position déployée.

6. Dispositif d'injection selon l'une quelconque des revendications précédentes, **caractérisé en ce que**, lorsque le protecteur d'aiguille (22) est dans la position déployée, le ou chaque crochet (56) est maintenu par coopération avec le support de protecteur (20) pour que le protecteur d'aiguille (22) ne puisse être ramené dans la position rétractée.

7. Dispositif d'injection comprenant, d'une part, une seringue (1) qui comprend une aiguille (6), un corps tubulaire (4) à l'extrémité distale duquel est fixée une aiguille (6), et un piston d'injection (8) monté à coulissement dans le corps (4) et, d'autre part, un dispositif (2) de protection d'aiguille selon l'une quelconque des revendications précédentes.

## Patentansprüche

1. Vorrichtung zum Schutz einer Nadel, aufweisend:
- einen Schutz-Halter (20), der einen Tunnel zur Aufnahme eines Spritzenkörpers (4) begrenzt,
einen Nadel-Schutz (22), der bezüglich des Schutz-Halters (20) bewegbar ist zwischen einer Einrückposition und einer Ausrückposition,
- eine Druckfeder (24), die zwischen dem Schutz-Halter (20) und dem Nadel-Schutz (22) eingesetzt ist,
wobei die Vorrichtung **gekennzeichnet ist durch**:
- wenigstens einen Ansatz (54) des Nadel-Schutzes (22) und wenigstens eine korrespondierende Nut (36) des Schutz-Halters (20), welche den besagten Ansatz aufnimmt und führt,
wobei die wenigstens eine Nut aufweist: einen ersten geradlinigen Abschnitt (38), welcher sich im Wesentlichen entlang einer Achse des Schutz-Halters (20) erstreckt über eine Länge, die größer ist als eine Länge der zu schützenden Nadel, und einen zweiten geradlinigen Abschnitt (40), der sich in einer schrägen Weise bezüglich der besagten Achse des Schutz-Halters (20) erstreckt, wobei der erste und der zweite Abschnitt (38, 40) eine V bilden, dessen Spitze zu einem proximalen Ende der Vorrichtung hin weist,
wobei der besagte wenigstens eine Ansatz (54) und die besagte wenigstens eine Nut (36) eingerichtet sind zum anfänglichen Rückhalten des Nadel-Schutzes (22) gegen die Wirkung der komprimierten Feder (24) in der Einrückposition, in welcher der wenigstens eine Ansatz (54) in dem zweiten geradlinigen Abschnitt (40) der wenigstens einen korrespondierenden Nut (36) rückgehalten ist,
wobei der Nadel-Schutz (22) freigebbar ist **durch** Verlagerung des Nadel-Schutzes (22) bezüglich des Schutz-Halters (22) entlang einer Freigebe-Richtung, was zur Verlagerung des wenigstens einen Ansatzes (54) in dem besagten zweiten geradlinigen Abschnitt (40) bis zu dem ersten geradlinigen Abschnitt (38) der wenigstens einen korrespondierenden Nut (36) führt,
und
- wenigstens einen Haltegreifer (56), der mit einem ersten von dem Schutz (22) und dem Halter (20) fest ausgebildet ist, wobei der oder jeder Haltegreifer verlagerbar ist zwischen einer Sperrposition in Anschlag gegen den zweiten von dem Schutz (22) und dem Halter (20) bei Abwesenheit einer Spritze in dem Schutz-Halter (20) und einer Entsperrposition im Abstand von dem zweiten von dem Schutz (22) und dem Halter (20) bei Anwesenheit einer Spritze in dem Schutz-Halter (20), wobei der oder jeder Haltegreifer (56) eine Wirkfläche (56B) hat, die mit dem Spritzenkörper (4) zusammenwirkt, wenn dieser in den Tunnel des Halters (20) eingreift, für das Passieren des Haltegreifers (56) von dessen Sperrposition in dessen Entsperrposition.

2. Vorrichtung zum Schutz einer Nadel gemäß Anspruch 1, **dadurch gekennzeichnet, dass** der oder jeder Haltegreifer (56) einen Vorsprung hat, der bei Abwesenheit des Spritzenkörpers (4) ins Innere des Tunnels des Schutz-Halters (20) vorsteht.

3. Vorrichtung zum Schutz einer Nadel gemäß irgendeinem der Ansprüche 1 und 2, **dadurch gekennzeichnet, dass** der oder jeder Haltegreifer (56) fest mit dem Nadel-Schutz (22) ausgebildet ist.

4. Vorrichtung zum Schutz einer Nadel gemäß Anspruch 3, **dadurch gekennzeichnet, dass** der oder jeder Haltegreifer (56) eine Mitnahmefläche (56B) hat und der Schutz-Halter (20) wenigstens eine Rampe (57) hat, die imstande ist, mit dem oder jeder Mitnahmefläche (56B) zusammenzuwirken zum Gewährleisten des Rückhaltens des oder jedes Haltegreifers (56) an der Außenseite des Schutz-Halters (20).

5. Vorrichtung zum Schutz einer Nadel gemäß irgendeinem der Ansprüche 1 bis 4, **dadurch gekennzeichnet, dass** der Schutz-Halter (20) eine distale Fläche (44B) aufweist, die imstande ist, mit dem oder jedem Haltegreifer (56) zusammenzuwirken zum Aufrechterhalten des Schutzes (20) in Ausrückposition.

6. Injektionsvorrichtung gemäß irgendeinem der vorhergehenden Ansprüche, **dadurch gekennzeichnet, dass**, wenn der Nadel-Schutz (22) in der Ausrückposition ist, der oder jeder Haltegreifer (56) in Zusammenwirkung mit dem Schutz-Halter (20) aufrechterhalten ist, damit der Nadel-Schutz (22) nicht in die Einrückposition rückführbar ist.

7. Injektionsvorrichtung aufweisend, einerseits eine Spritze (1), die eine Nadel (6), einen rohrförmigen Körper (4), an dessen distalen Ende eine Nadel (6) befestigt ist, und einen Injektionskolben (8) aufweist, der verschiebbar in dem Körper (4) montiert ist, und andererseits eine Vorrichtung (2) zum Schutz einer Nadel gemäß irgendeinem der vorhergehenden Ansprüche.

## Claims

1. Needle protection device comprising:
- a protective support (20) delimiting a tube for receiving a syringe body (4);
- a needle protector (22) movable in relation to the protective support (20) between a retracted position and a deployed position;
- a compression spring (24) applied between the protective support (20) and the needle protector (22);
said device being **characterised by**:
- at least one pin (54) of the needle protector (22) and at least one corresponding groove (36) of the protective support (20), receiving and guiding said pin,
the at least one groove comprising: a first rectilinear part (38) which extends substantially along an axis of the protective support (20) over a length greater than the length of the needle to be protected; and a second rectilinear part (40) that extends inclined in relation to said axis of the protective support (20), the first and second parts (38, 40) forming a V, one point of which is directed towards a proximal end of the device,
said at least one pin (54) and said at least one groove (36) being configured for the initial retention of the needle protector (22) against the action of the compressed spring (24) in the retracted position, in which the at least one pin (54) is retained in the second rectilinear part (40) of the at least one corresponding groove (36),
the needle protector (22) being releasable by a displacement of the needle protector (22) in relation to the protective support (20) along a releasing direction, leading to the displacement of the at least one pin (54) in said second rectilinear part (40) up to the first rectilinear part (38) of the at least one corresponding groove (36);
and
- at least one bracket (56) integral with a first of the protector (22) and of the support (20), the or each bracket being movable between a blocking position stopped against the second of the protector (22) and of the support (20) in the absence of a syringe in the protective support (20) and an unblocking position separated from the second of the protector (22) and of the support (20) in the presence of a syringe in the protective support (20), the or each bracket (56) having an actuating face (56B) that cooperates with the syringe body (4) during its engagement in the tube of the support (20) for the transitioning of the bracket (56) from its blocking position to its unblocking position.

2. Needle protection device according to claim 1, **characterised in that** the or each bracket (56) has a protuberance projecting inside the tube of the protective support (20) in the absence of the syringe body (4).

3. Needle protection device according to either one of claims 1 or 2, **characterised in that** the or each bracket (56) is integral with the needle protector (22).

4. Needle protection device according to claim 3, **characterised in that** the or each bracket (56) has a cam surface (56B) and the protective support (20) comprises at least one ramp (57) capable of cooperating with the or each cam surface (56B) to ensure the elimination of the or each bracket (56) outside the protective support (20).

5. Needle protection device according to any one of claims 1 to 4, **characterised in that** the protective support (20) comprises a distal surface (44B) capable of cooperating with the or each bracket (56) to maintain the protector (20) in the deployed position.

6. Injection device according to any one of the preceding claims, **characterised in that**, when the needle protector (22) is in the deployed position, the or each bracket (56) is held by cooperation with the protective support (20) so that the needle protector (22) cannot be returned to the retracted position.

7. Injection device comprising, on the one hand, a syringe (1) that comprises a needle (6), a tubular body (4) at the distal end of which a needle (6) is attached, and an injection piston (8) mounted capable of sliding inside the body (4) and, on the other hand, a needle protection device (2) according to any one of the preceding claims.
